# EUROPEAN PATENT SPECIFICATION

(11) **EP 1 663 069 B1**
(45) Date of publication and mention of the grant of the patent: **06.02.2019**
(21) Application number: 04765548.5
(22) Date of filing: 23.09.2004
(51) Int. Cl.: A61F 2/95, B29C 61/00, B29L 31/00, B29C 61/02, A61F 2/82

(54) **IMPLANT WITH SHAPE MEMORY**
IMPLANTAT MIT FORMGEDÄCHTNIS
IMPLANT A MEMOIRE DE FORME

(30) Priority: 23.09.2003 GB 0322286
(43) Date of publication of application: 07.06.2006
(73) Proprietor: Angiomed GmbH & Co. Medizintechnik KG, 76227 Karlsruhe (DE)
(72) Inventor: BLANK, Thiemo, 68723 Plankstadt (DE); PATHAK, Chandrashekhar, Prabhakar, Phoenix, Arizona 85048 (US)
(74) Representative: Hoffmann Eitle
(86) International application number: PCT/EP2004/010692
(87) International publication number: WO 2005/027792

(56) References cited:
- EP-A- 0 823 245
- US-A1- 2003 055 198
- US-B1- 6 176 871
- US-B1- 6 533 752
- US-B1- 6 607 551

## Description

This invention relates to implants, to be implanted in the human or animal body. More particularly, but not exclusively, it concerns tubular grafts, devices for stenting bodily lumens, and devices such as filters to be installed within a bodily lumen.

### Background prior art

Devices for stenting bodily lumens can be categorised as non-expanding stents, balloon-expandable stents or self-expanding stents. They are made of biologically compatible material and, until now, it has been customary to use a metal as the material of construction of any expanding stent, in order that it shall have sufficient strength to expand radially outwardly and maintain the bodily lumen patent after its placement at a stenting site by a suitable delivery catheter. Non-expanding stents, by contrast, are often metal-free grafts and are placed at a prosthesis site in the body of the patient during invasive or open surgery.

For minimally invasive procedures, stents are delivered on the distal end of a catheter which is introduced transluminally and often also percutaneously, as by the Seldinger procedure via the iliac artery. Balloon-expandable stents are usually made of stainless steel and placed around a generally cylindrical inflatable balloon which lies along the lumen of the stent matrix. At the stenting site, the balloon is inflated, causing plastic deformation of the material of the stent matrix, and radial expansion of the matrix to urge the bodily tissue forming the wall of the lumen in a radially outward direction. With deflation of the balloon, the delivery system can be withdrawn transluminally from the lumen of the expanded stent leaving the plastically deformed stent in place.

Conversely, self-expanding stents are delivered on the distal end of a catheter which often features a sheath surrounding the radially compressed stent matrix. Upon proximal withdrawal of the sheath relative to the stent matrix, the stent matrix can self-expand radially, progressively from one end of the stent matrix to the other, as the sheath is withdrawn axially along and relative to the radially outside surface of the stent matrix.

Once such a stent has been placed, and the delivery system withdrawn, it is difficult, or indeed impossible, to remove the stent, other than perhaps by open surgery. There have been many proposals for recovering stents after the delivery system has been parted from the expanded stent, but what degree of success any of these proposals have enjoyed is unclear. See, for example, WO 03/049691 for a stent of polymer and metal, which is delivered percutaneously with a small radial diameter, then expanded at a stenting site and the catheter-based delivery system removed. Removal of the stent is not discussed or contemplated. The device of US-A-5716410 is removable, but here the device is a catheter with a distal end that performs a dilating function. Although the device is said to perform a "stenting" function, it is not an implant. Its functionality is like that of a balloon catheter rather than that of a stent.

There are many clinical situations in which it would be desirable to use an implant to support or occlude a bodily lumen in one deployed configuration and then, when the indication for such intervention changes, to change the diameter of the implant, either to change to a different deployed configuration, or to a withdrawal configuration.

If a way could be found to reduce substantially the diameter of an implant in situ, then the implant might be transluminally removable from the body. There are also situations in which it would be useful, in successive surgical interventions, to increase step-wise with each intervention the diameter of an implant.

US-A-6,176,871 discloses a catheter for photothermoforming a stent, and a photothermoformable stent. Within the lumen of a catheter, surrounded by a stent-expanding balloon, is a diffuser 10 that emits light radially outwardly through the balloon and into the bulk volume of a stent matrix carried on the balloon, where it can be absorbed by a chromophore distributed throughout said bulk, for raising the temperature of the stent, to enable it to be molded by the balloon, as it inflates, against the bodily tissue wall of the lumen into which the stent has been advanced.

The reader is hereby referred to the entirety of US-A-6,176,871 for the technical teaching contained therein.

EP0823245 discloses a system comprising a stent and a balloon catheter wherein the stent can be removed by once again concentrically positioning the balloon catheter relatively to the previously deployed stent at the treatment site, heat the balloon to apply heat to the stent until it reaches its transition temperature and begins to return to its first memorized, reduced first diameter.

### Summary of the invention

According to the present invention, there is provided a system comprising: an implant for implantation at an implantation site within a lumen of the body of a human or animal, the implant having a matrix with a luminal surface, an abluminal surface and a wall thickness separating said surfaces, the implant having capability to expand and contract radially through a range of diameters, with corresponding changes in the cross-sectional area of the lumen defined by the matrix, said implant exhibiting a remembered configuration in which the diameter of the implant is at the low end of said range of diameters, such that the implant is prompted to change its configuration towards said remembered configuration, that is, towards a reduced diameter configuration, upon the imposition on the implant of outside stimulus, and said implant being characterised by a molecule distributed within its bulk volume that preferentially responds to an applied field that permeates said volume by absorbing energy at a particular wavelength from said applied field, wherein said applied field constitutes the outside stimulus, and the preferential response of the molecule raises the temperature of the implant, the temperature rise serving as the prompt on the implant to change to said remembered configuration; and a self-shrinkage activation catheter equipped to impose on the implant said applied field thereby to stimulate the implant to contract radially from a first deployed configuration towards its remembered configuration, wherein the activation catheter is a balloon catheter with means to manage the temperature of the surface of the balloon, whereby said balloon surface is effective to conduct heat energy to the luminal surface of the implant matrix.

This is the realisation of a system for delivery and/or retrieval and/or translocation of an implant for a bodily lumen which has a self-shrinking capability activated by an effective flux from an externally applied field. The present invention embraces the concept of "shaping" the implant at temperatures above body temperatures, with the material of the implant being shape-stable or "frozen" at body temperature, but is not restricted to such a concept. For example, it is envisaged to use a molecule that switches from one configuration to another when exposed to the field, the switch of configuration delivering the shape change that will bring the implant to the smaller diameter configuration that is desired.

The concept of a "field" in this specification includes not only static and varying (such as alternating) magnetic and electric fields but also beams of electromagnetic radiation of specified wavelength and beams of particles. The flux from the field can also be another energy-bearing flux such as of ultrasound energy.

The concept of a "self-expanding" stent is well understood. What is meant by a "self-shrinking" capability is a capability of the prosthesis itself to reduce on command its transverse radial dimension so as to move, of its own volition, when required, from a relatively large radius disposition to a relatively smaller radius disposition. In one example, a stent or graft could be induced to shrink radially. In another example a filter within an artery, such as the carotid or vena cava artery, could be induced to shrink radially, such shrinkage in both cases even perhaps allowing transluminal removal of the prosthesis from the body.

A self-shrinking capability can be provided by selecting as the structural material of the prosthesis a shape memory polymer.

For sufficient information to realise the present invention with a shape memory polymer, attention is hereby directed to the patent publications of Mnemoscience GmbH, amongst which are: WO 99/42147, WO 99/42528, WO 01/91822 and WO 02/083786, as well as US patent publications 6160084, 6388043B and 2003-0055198A1. It is within the present inventive concepts, in particular, to provide implants of biodegradable material, and implants that serve as delivery vehicles for substances and compositions that are biologically useful such as medicaments.

A self-shrinking capability in a structure of shape memory polymer is accomplished by giving the structure a memory of a radially relatively small disposition. At the site of implantation, the implant is used to perform a function, at its relatively large radial disposition. When it is desired to remove the implant from the body, or to reduce its diameter yet leave it in place. This can be done, for example, by changing the temperature of the structure relative to body temperature, that is to say, for example by heating the implant above body temperature, to trigger the memory, that is, to give the structure a thermodynamic driving force sufficient for the implant to change its shape towards the remembered small radius configuration.

To maintain a temperature differential between the structure and ambient body temperature, one can provide within a lumen of the implant a thin wall balloon inflated with liquid medium that delivers a sufficient conductive thermal flow to the implant structure, and then arrange for the volume of the balloon, and its cylindrical cross-section, to be reduced sufficiently gradually for the structure to follow the radial shrinking so that the implant remains in contact with the balloon surface and with the conductive thermal flow from within the balloon.

It will be appreciated that the technical feature which decisively distinguishes a stent matrix which is a self-shrinker from one which is a self-expander is that the remembered diameter is at or below the small radius transport disposition of the stent matrix rather than the large diameter stenting configuration. Whereas a self-expanding stent at bodily temperature is inclined to expand radially outwardly, a self-shrinker is "programmed" to move (at a selected temperature which might not be body temperature) towards a small radius disposition.

Nevertheless, one may envisage a prosthesis which can be both a self-expander and a self-shrinker, with a first imposed stimulus, such as a proximal withdrawal of a sheath that surrounds and confines the implant, stimulating or permitting an elastic deformation that delivers a desired radial expansion. Then, a second stimulus, namely the said externally applied field, serves to stimulate a return to a remembered smaller radius disposition, to enable removal of the implant (or a shift from a first deployed configuration to a second deployed configuration).

With shape memory polymers, in contradiction to shape memory alloys, an input of energy is needed in order to permit the adjustments of long chain molecules which are needed in order for the bulk polymer to revert to its remembered configuration. In the case of a nickel-titanium shape memory alloy stent, raising its temperature above body temperature simply increases the thermodynamic driving force from the martensitic to the austenitic configuration, thereby increasing the force with which the stent urges the tissue forming the wall of the stenting lumen radially outwardly. By contrast, with a shape memory polymer as contemplated here, it can be arranged that raising the temperature of the polymer stent significantly above body temperature has the opposite effect, warming the bulk polymer sufficiently to liberate relative movements of molecular chains and thereby permit reversion to the remembered configuration. At body temperature, by contrast, it may be arranged that reversion to the remembered shape is frustrated by the "freezing" at that body temperature, of the positions of the molecular chains, relative to each other.

EP-A-823 245 discloses a retrievable shape memory stent which responds to heating above body temperature by reverting to a small diameter configuration. By introducing into the lumen of the deployed stent the balloon of a balloon catheter, then inflating the balloon with hot liquid that by conduction of heat through the balloon wall warms the material of the stent, the stent is brought to a temperature which enables it to move towards its small diameter "remembered" configuration.

By deflating the balloon slowly enough to maintain by thermal conduction a flux of heat energy from the balloon to the stent that is large enough to maintain the stent temperature sufficiently above body temperature, the stent can be returned on the balloon out of the body. But the magnitude of the thermal flux from balloon to stent is a conductive flow, and depends on the quality of the conductive path from the liquid within the balloon to the bulk of the polymer of the stent. This quality is relatively good when the balloon is being increased in size, to push the stent from small to large diameter. This quality is less good, when the balloon is being reduced in size so as all the time to be moving away from the luminal wall surface of the stent. If the hot balloon shrinks too fast, the stent will be cooled back towards body temperature by the bodily tissue pressing on its abluminal surface, and by the bodily fluids in the lumen it is stenting. Any such cooling will "freeze" the stent at its instantaneous diameter, thereby frustrating attempts to bring its diameter down to one small enough for its transluminal removal.

Especially in cases where tissue has grown over and around an implant to be removed, getting the implant away from the tissue may be difficult. A higher driving force can be generated by increasing the difference of temperature between the implant and adjacent tissue, but this runs the risk of tissue damage from over-heating.

By contrast, using an external field, such as flooding the implant with light at a particular wavelength, to stimulate a molecule in the bulk polymer of the stent (such as a chromophore) eliminates any dependency on the establishment and maintenance of a thermally conductive path from the liquid in the balloon to the polymer of the stent. Instead, with an energy flux to the chromophore (or other molecule) derived from a field of, say, electromagnetic radiation, energy reaches the polymer of the stent in full measure regardless of the instant dimensions of any balloon on the stent retrieval catheter. In this way, the energy flux or other field can be fully effective in minimum time, thereby also minimising any unwanted and damaging heating of bodily tissue or fluids by the recovery catheter. Specifically, if the balloon is deflated faster than the stent can shrink, the energy flux from an imposed field is still received in undiminished amount by the polymer of the stent, thereby reducing the risk that the stent is prematurely cooled towards body temperature and the attendant risk that the stent stops shrinking before its diameter is down to the desired small size.

By now, there is a wealth of experience in the technical field of stent design, and an equal wealth of experience in the technical field of balloon catheters. Accordingly, putting the present invention into effect should be within the capability of a team of skilled individuals which includes a polymer scientist familiar with the state of the art in shape memory polymers, a stent designer and a balloon catheter designer.

Those skilled in the art of stenting, especially balloon expandable stents, have considerable and detailed experience of management of fluids within the balloons of catheters used for placing balloon expandable stents at stenting sites. It is within the capability of such individuals to manage flow of liquid through the balloon of the catheter in order to achieve the controlled rate of balloon deflation mentioned above.

Those skilled in the art of shape memory polymers will be able to think of alternative ways of managing an energy flux or other field sufficient to trigger the shape memory effect and the phenomenon of shrinkage of the radial dimension of the stent, to permit withdrawal of the stent from the body. One envisages the use of electromagnetic radiation, transmitted from the distal end of a retrieval catheter, to issue from the catheter over the length of the stent and around the full circumference, so that all portions of the luminal wall of the stent are bathed in the radiation, whereby all portions of the bulk material of the stent are exposed to the effect of such radiation.

Alternatively, one envisages instead of electromagnetic radiation a discharge of molecular, atomic, or sub-atomic particles from the distal end of a retrieval catheter into the bulk of the stent via its luminal wall surface.

Alternatively, one envisages the use of a bodily fluid filling the lumen of the stent as a means of transmission of the energy flux from the distal end of the retrieval catheter into the bulk material of the stent. One thinks of, for example, the generation of ultrasonic vibrational energy in the distal end, transmitted to the stent via the bodily fluid in the lumen. Otherwise, static or varying electric or magnetic fields can be delivered to the implant from a catheter within its lumen, with the field serving to stimulate a change in the molecule such as a switch from one configuration or orientation to another.

Not out of the question is the use of energy-emitting devices wholly outside the body but which are capable of directing an energy flux or other field through any intervening bodily tissue to focus on the matrix of the implant which reacts to the field. Ultrasound and electromagnetic radiation (at least) are credible as such fields.

Catheter delivery systems, after decades of development, are by now flexible and sophisticated. Those skilled in the art of designing such delivery systems will not find it difficult to adapt them to the delivery of a prosthesis in accordance with the present invention. As to retrieval systems, they will resemble delivery systems to the extent that they both have the task of transporting a prosthesis between a point of bodily entry and a point of placement of the prosthesis. It is simply that the path of movement of the prosthesis is reversed.

As to management of energy flux, present day stent delivery systems are required to achieve an energy input at the distal end, at the stenting site, for example, by inflating a balloon or by withdrawing a sheath against the frictional forces of a self-expanding stent within the sheath and pressing on the luminal wall of the sheath. Management of energy flux for the retrieval systems contemplated in the present invention is a step further, but is not an unknown field. For example, a well-established field of catheter development which is likely to prove useful in the context of the present invention is the field of catheters for electrical stimulation of tissue on the wall of a chamber of the heart. One class of these catheters seeks to traumatise by electrical energy particular confined areas of tissue in order to extinguish aberrant electrical signals within the bodily tissue of the heart which cause the heart to beat erratically. Another class of such catheters uses laser energy to ablate tissue. Clearly, such catheters are required to deliver a flux of energy out of the cylindrical wall of the distal tip of the catheter. Such an energy flux which is contemplated, in the present invention, for stimulating shrinkage of the present implant from their radially large disposition to their radially smaller withdrawal disposition.

Evidently, applications of self-shrinking shape memory polymer stents that involve a relatively short length and large diameter delivery system will be favoured over those which require notably long and thin delivery systems. One example that springs immediately into mind is temporary stenting of the oesophagus or trachea. Other interesting possibilities are the bile duct, the uro-genital tract and the gastro-intestinal tract so that applications may extend to: vascular, coronary, urological, oesophageal, gastro-intestinal, biliary, colo-rectal, duodenal, tracheobronchial, pulmonary, vaginal and pancreatic. Biliary applications are viewed with particular interest.

Another advantage of stenting a bodily lumen that is large in diameter is that a relatively large wall thickness for the shape memory polymer implant can be contemplated. The ability of the shape memory polymer to maintain bodily tissue radially outwardly against pressure imposed on the implant radially inwardly by the tissue may be significantly different from the performance of stents made of metal. This may be another reason for pioneering shape memory polymer implants in bodily lumens of most ready access. Besides the trachea or oesophagus, another possibility of interest is the colo-rectal area.

### Brief description of the drawings

For a better understanding of the present invention and to show more clearly how the same may be carried into effect, reference will now be made, by way of example, to the accompanying drawings, in which:
- Figs. 1 to 6: are all longitudinal diametral sections through a stenosis in a bodily lumen, and show successive stages of surgical intervention, with an implant, to ameliorate the stenosis.
- Fig. 7: is a longitudinal section through the colon and through a retrieval catheter.

### Detailed description

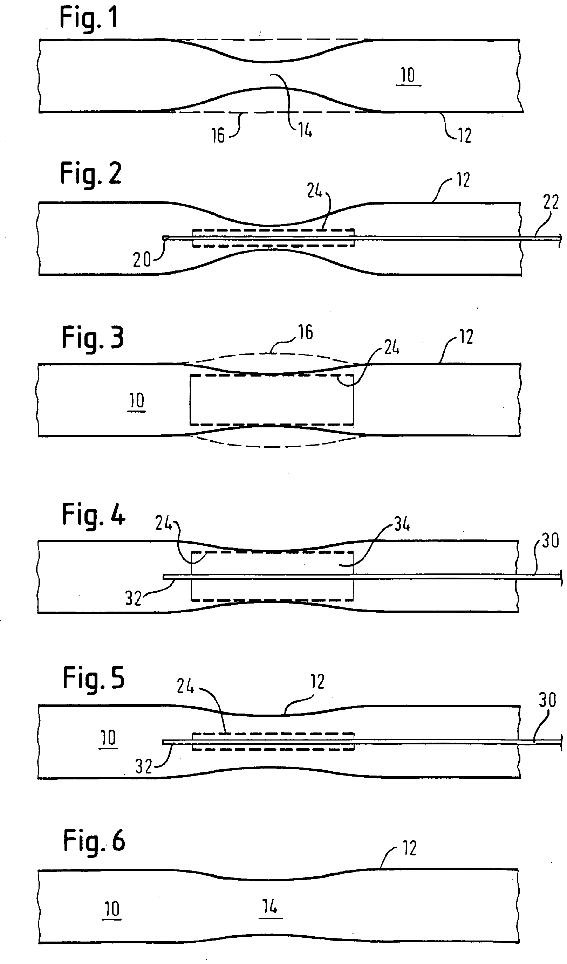

Referring to the drawings, Fig. 1 shows in diametral longitudinal section part of a bodily lumen which may be a lumen 10 defined by a lumen wall 12 of bodily tissue that is abnormal at a stenting site 14 in the lumen 10 where the diameter of the lumen 10 is restricted by ingress into the lumen of unwanted bodily tissue 16.

Fig. 2 shows the same part of the same lumen, but also the distal end 20 of a catheter 22 that carries at its distal end a stent 24 which is being employed to maintain patency within the stenting site 14. As can be seen, the stent 24 has for the time being a relatively small diameter, permitting it to be advanced into the narrow part 14 of the lumen 10, so that it extends across the narrow part, ready for radial expansion.

Fig. 3 shows again the same portion of the same lumen, but with the stent 24 already expanded radially outwardly from its small radius delivery disposition of Fig. 2 into its larger deployed stenting radius in Fig. 3 for holding back radially outwardly the tissue of the lumen wall 12 and tissue 16. In Fig. 3, the delivery catheter 22 has been withdrawn proximally in the direction reverse to that in which its distal end was advanced into the lumen. Thus, with the delivery system withdrawn from the body, the stent 24 is left behind an implant within the body, to maintain patency along the lumen 10.

Fig. 4 shows again the same location in the same lumen, but also a stent retrieval catheter 30 having been advanced so that its distal end 32 has been advanced through the lumen 34 of the expanded stent. In Fig. 4, the retrieval catheter 30 is shown only schematically. Lacking from the drawing Figure are the technical features at the distal end of the catheter 30 that interact with the stent 24, once the distal end of the catheter 32 has been advanced through the lumen 34 of the stent 24. These features will be explained below.

Moving to Fig. 5, we see here the same site but the stent 24 has been caused to reduce its radial dimension, back towards a dimension similar or the same as that shown in Fig. 2 that was characteristic of its disposition for delivery to the stenting site. The shift of stent radial dimension from the stenting disposition of Fig. 4 to the retrieval disposition of Fig. 5, is accomplished by causing a flux of energy (explained below) to flow between the retrieval catheter 30 and the stent 24. Contraction of the stent in the radial direction brings the stent 24 into engagement with the distal end 32 of the retrieval catheter system 30, and out of engagement with the wall 12 of the lumen 10.

Finally, in Fig. 6, we see the same bodily lumen 10 and wall surfaces 12, but the zone 14 that was previously narrowed (Fig. 1) is now less narrow, as a result of the temporary occupation of the narrow zone by the stent 24. With the greater diameter of the zone 14, the lumen can function satisfactorily or adequately, without the continuing presence of the stent 24.

Within the state of the art, a stent, once placed, remains permanently at the stenting site, and there is no provision, transluminally to remove the stent, once placed. Thus, when the placement procedure is defective, it may be that open surgery is needed to rectify the defective placement. It would be advantageous to offer medical practitioners stents that are more "forgiving", in the sense that they can be moved again, for example after defective placement, to a correct position axially displaced along the bodily lumen from the incorrect placement.

Even if the implant is placed correctly, there may still be clinical reasons to want to move it subsequently to a different position in the bodily lumen, that is, to translocate it. The invention opens up possibilities to do this.

However, there are medical conditions in which the body needs the assistance of a stent only temporarily. In one example, one might wish to support temporarily colo-rectal bodily tissue immediately adjacent to an end-to-end anastomosis of the colon. In such instances, it may be beneficial for the patient to be able to perform an after-procedure, once the implant has proved effective, to remove the implant from the healed site.

It is in such instances that the present invention is attractive. The implant of the present invention is characterised over prior art stents in that it is responsive to an energy flux to shift of its own volition from a radially large stenting disposition to a radially smaller withdrawal disposition (the transition shown schematically in moving from Fig. 4 to Fig. 5).

Those skilled in the art of shape memory polymers are aware of various mechanisms by which a cylindrical lattice or mesh of shape memory polymer, whether made out of tube, sheet or wire, and serving as a stent, could be persuaded to shift of its own volition to a smaller radius disposition.

Coming most immediately to mind, as a stimulus to shift the radial dimension downwards, is the imposition of a change of temperature on the shape memory polymer lattice material. Thus, for example, one envisages introducing into the lumen of the shape memory polymer stent its stenting disposition of Fig. 4, the distal end of a retrieval catheter which has the capability of delivering a thermal flux to the luminal wall of the lattice of the polymer of the stent 24, thereby warming the lattice above body temperature and thereby triggering an inclination within the lattice to revert to the small diameter remembered configuration. Various possibilities will occur to those skilled in the art how to engineer a thermal flux from the retrieval system 30 to the bulk material of the stent 24. Thermal energy could be radiated from the cylindrical surface of the distal end 32 of the retrieval catheter 30. Additional thermal flux can also be delivered to the stent 24 by direct surface to surface thermal conduction. For example, the distal end 32 of the retrieval catheter could be in the form of an inflatable balloon with a cylindrical outer surface adapted to be inflated into the face-to-face contact with the luminal surface of the stent 24. By flowing heated liquid (or conceivably gas) through the interior chamber of the balloon, one would rely on thermal flux radially outwardly through the wall thickness of the balloon membrane, and thereby into the bulk of the lattice of the stent 24 with which the radially outer surface of the balloon membrane is in face-to-face contact.

Having warmed the stent 24 to a temperature above body temperature, and high enough to permit the polymer of the stent to move of its own volition towards the remembered small diameter configuration that it has previously been given, one would then progressively and gradually arrange for the volume and radial dimension of the heated balloon to decrease, and it would be the tendency of the polymer of the stent 24 to follow down the reducing radial dimension of the balloon so that, when the radial dimension of the balloon is desirably reduced, or small enough to permit withdrawal out of the body of the distal end 32 of the retrieval system 30, the stent 24 is still in face-to-face contact with the deflated balloon, at a small radial dimension, and ready to be withdrawn from the body, carried on the distal end of the retrieval system 30, as shown in Figs. 5 and 6.

Reverting to Fig. 5, once the stent 24 has been "encouraged" to move towards a small diameter configuration, and before the shrunken stent is withdrawn from the body, one envisages taking steps to make the shrunken stent 24 fast on the distal end 32 of the retrieval system 30. One way to accomplish this would be to cause the stent 24 to shrink down onto surface formations of the distal end 32 that in some way engage with surfaces of the stent 24 and prevent any tendency of the shrunken stent 24 to move distally (that is to say, from right to left in Fig. 5) relative to the retrieval system 30. One envisages abutment surfaces, hooks or reentrant surfaces on the distal end 32 to engage with one point or another of the stent lattice 24.

Alternatively, one could advance along the line of the retrieval system 30 a sheath element into which the shrunken stent 24 can be drawn, or pushed, in preparation for its journey along the bodily lumen, from the stenting site of the drawing Figures to the point of percutaneous entry to the body.

Other possibilities will occur to skilled readers. For example, there are some bodily lumens where percutaneous entry is not required. One example is lumens of the urinary tract. Another example is the gastro-intestinal tract. The present invention is equally applicable to bodily lumens where no percutaneous entry point is needed.

In the discussion above, the lumen was taken to be that of the colon. The present invention is as much applicable to any other accessible lumen of the body.

In the decades since the first metal stent was proposed, there has been rapid and copious development of innumerable different strut designs for stents. For the design of stents in accordance with the present invention, all of this design knowledge is available, for what it is worth. Each material has its own characteristics and capabilities, which determine what will be its mechanical characteristics and what forming techniques and possibilities are feasible. Clearly, successful development of effective shape memory polymer stents will be facilitated by putting together in one team a person specialising in shape memory polymers, and another person specialising in the design of stents, as such. Such a team will be able to identify from the portfolio of design literature of stents hitherto (predominantly metal, notably stainless steel and nickel-titanium shape memory alloy) those designs which are of interest to the person seeking to make a stent out of shape memory polymer.

Nevertheless, a new range of design possibilities is opened up, by moving from metals to polymers. For example, one envisages designs featuring continuous film instead of meshes with struts and interstices. In this way, the implants contemplated here could serve not just as a stent but also as a stent graft.

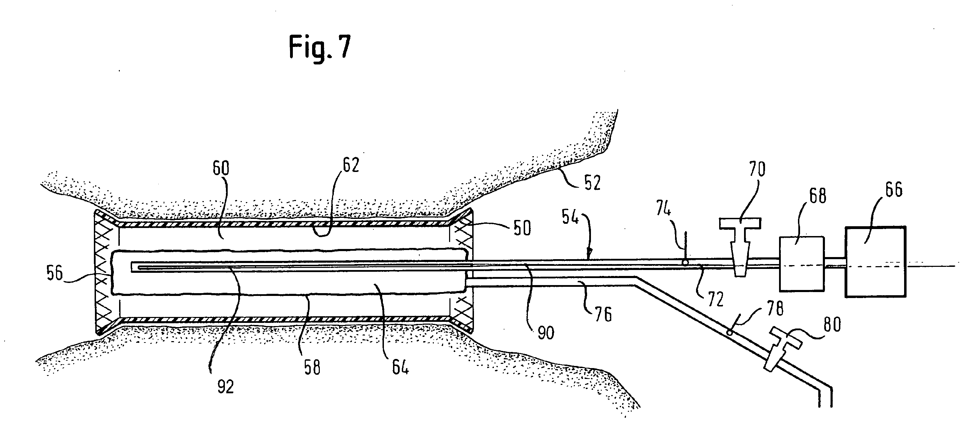

Referring now to Fig. 7, a stent graft 50 is installed within the colon 52 of a patient and is to be removed using a retrieval catheter system 54. The stent grant 50 has a structural matrix of shape memory polymer as described above, coated with graft material that contains medication or other biologically active material.

For removal of the stent graft 50 from the colon'52, a retrieval catheter system 54 is selected, which has a distal end 56 for advancing into the colon and into the lumen of the stent graft 50. In a distal end zone of the catheter is an inflation balloon defined by a balloon membrane 58, this balloon having a length sufficiently great to interact with the shape memory polymer structure of the stent graft over substantially its full length.

In a deflated condition of the balloon, the membrane passes easily and with clearance through the lumen 60 defined by the stent graft. Once the balloon is in position within this lumen, fluid is introduced into the balloon cavity, thereby inflating the balloon and bringing the balloon membrane 58 into pressure contact with the luminal surface 62 of the stent graft 50. With this contact, a thermal flux can be engineered, as in EP-A-823245 (mentioned above) between the fluid in the balloon cavity and the structural material of the stent graft. This thermal flux can raise the temperature of the implant above body temperature, and thereby "liberate" the ability of the shape memory polymer to revert to its remembered small radius configuration. Thus, once the shape memory polymer has been stimulated by the thermal flux to shrink, one reduces the volume and cross-sectional area of the inflated balloon, progressively, while aiming to maintain the thermal flux through the balloon membrane 58, and the pressure contact of the shrinking stent graft with the balloon membrane 58, so that the shrinking stent graft follows radially downwards the shrinking cross-sectional dimension of the balloon 58.

With the stent graft 50 shrunk down radially to the size of the deflated balloon in Fig 7, it will be evident that the stent graft, still on the balloon 58, can then be withdrawn from the colon.

In a variant, the diameter of the implant could be reduced, using the same technique, to any diameter smaller than its deployed diameter hitherto, and then left in place for a further period of time.

Hot liquid for the balloon cavity 64 can be provided from a reservoir 66 which feeds a pump 68 which generates a head of pressure sufficient to inflate the balloon as required. Flow control means 70, downstream of the pump 68, can be controlled by a microprocessor (not shown) to admit to balloon in-feed lumen 72 a sufficient flow of heated fluid. An upstream temperature sensor 74 monitors the temperature of the fluid in the in-feed lumen 72 and the monitored temperature provides another data in-feed to the microprocessor control (not shown).

The in-feed lumen 72 extends uninterrupted to the distal end of the balloon cavity 64, a lumen 76 for fluid venting of the balloon cavity 64 being located at the opposite end of the length of the balloon cavity 64, so that fluid entering the balloon cavity 64 must flow the entire length of the balloon cavity before it may leave the cavity. More refined or effective heat exchange flow path arrangements will be evident to those skilled in the art of heat exchange. Otherwise, heat or some other energy flux could be generated by initiating a chemical reaction (such as an exothermic reaction) at the location of the implant.

Fluid leaving the balloon cavity 64 in the exhaust lumen 76 flows past a downstream temperature sensor 78, and then past a flow controller 80 which governs the rate of release of fluid from the balloon cavity 64. The microprocessor control coordinates the in-feed pressure and input and output flow rates to and from the balloon cavity 64 so as to maintain in the balloon cavity for both a programmed temperature of the balloon membrane 58 and a programmed inflation and slow deflation of the balloon volume thereby to engage as desired with the stent graft 50, initiate self-shrinkage capability, follow the shrinkage down, and then withdraw the shrunken stent graft on the deflated balloon 58.

Figure 7 also shows within fluid infeed lumen 72 an optical fibre 90 to a diffuser 92 which diffuses radially outwardly through the balloon membrane 58 electromagnetic radiation which is chosen to complement the light absorption characteristics of a chromophore evenly distributed throughout the bulk of the polymer material of which the stent 24 is formed. Thus, beaming light along the optical fibre 90 to the diffuser 92 heats the polymer and raises its temperature sufficiently above body temperature to trigger its radial shrinkage.

Thus, Fig. 7 shows two separate and distinct means to trigger the self-shrinking behaviour of the implant, namely, conductive flow of heat from the fluid in the balloon, and subjecting the implant to an externally applied field (here light). We envisage using just the field and managing without any conductive heat flow to the implant, whenever the response of the implant to the field is strong enough to permit this.

Having the stent shrink while the balloon membrane 58 is in contact with its abluminal surface offers the possibilities at least of
i supplementary conductive heating of the stent polymer (as described above and in EP-A-823245)
ii pinching of balloon membrane 58 material within slots or through apertures of diminishing width as the stent shrinks radially. Such pinching could leave the shrunken stent matrix bound to the balloon, whereby trans-luminally withdrawing the balloon catheter from the illustrated bodily lumen more or less reliably carries the stent out too, without resort to supplementary devices to clamp, the shrunken stent to the catheter.

Above-mentioned US-A-6,176,871 is a reservoir of disclosure to enable useful choices of chromophores and complementary radiation frequencies to be made.

Since the stent is to be retrieved the chromophores do not remain in the body indefinitely, easing Government regulatory approval issues for such stents.

Although the illustrated embodiments are of stents and stent grafts, it will be appreciated that the present invention has wider application. Whereas stents and stent grafts are normally installed and not later removed, there are implants such as protection filters for temporary installation into the carotid artery when stenting the carotid, that are not left in situ and are removed after placement of the stent. Yet another field of application, in which a self-shrinking implant could be useful, is the field of ports and drainage systems exhibiting first and second deployment configurations, for example, "open" and "closed" and an energy flux controller to move the part or drainage device in a predetermined way, or on command, between the first and second configurations. Clearly, the provision of a filter (or any other device that temporarily occludes a bodily lumen) in the form of a "self-shrinker" in accordance with the present invention will bring substantial advantages in surgery. The present invention is not restricted therefore to implants which are stents and stent grafts. The illustrated embodiments provide the skilled reader with teaching how to realise particular individual devices within the scope of the claims which follow. It is to be understood that the scope of the claims is not limited to any feature of any of the illustrated embodiments. Further, it is to be understood that the skilled reader will take individual features from individual illustrated embodiments and put together other technical feature combinations to the extent that these are compatible and consistent with the teaching above.

## Claims

1. A system comprising:
an implant for implantation at an implantation site within a lumen of the body of a human or animal, the implant (24, 62) having a matrix with a luminal surface, an abluminal surface and a wall thickness separating said surfaces, the implant having capability to expand and contract radially through a range of diameters, with corresponding changes in the cross-sectional area of the lumen (34, 60) defined by the matrix,
said implant exhibiting a remembered configuration in which the diameter of the implant is at the low end of said range of diameters, such that the implant is prompted to change its configuration towards said remembered configuration, that is, towards a reduced diameter configuration, upon the imposition on the implant of outside stimulus, and
said implant being **characterised by** a molecule distributed within its bulk volume that preferentially responds to an applied field that permeates said volume by absorbing energy at a particular wavelength from said applied field, wherein said applied field constitutes the outside stimulus, and the preferential response of the molecule raises the temperature of the implant, the temperature rise serving as the prompt on the implant to change to said remembered configuration; and
a self-shrinkage activation catheter (32, 54) equipped to impose on the implant said applied field thereby to stimulate the implant to contract radially from a first deployed configuration towards its remembered configuration,
wherein the activation catheter (32, 54) is a balloon catheter with means to manage the temperature of the surface of the balloon, whereby said balloon surface is effective to conduct heat energy to the luminal surface of the implant matrix.

2. The system as claimed in claim 1, wherein said molecule is a chromophore.

3. The system as claimed in claim 1, wherein the applied field prompts said molecule to switch from one configuration to another configuration.

4. The system as claimed in any one of the preceding claims, wherein in the implant is made of a shape memory polymer.

5. The system as claimed in any one of the preceding claims, in which the implant is a stent.

6. The system as claimed in any one of the preceding claims, wherein the activation catheter (32, 54) is equipped with means to expand the implant radially outwardly to a second deployed configuration distinguished by an implant diameter greater than that of the first deployed configuration.

7. The system as claimed in any one of the preceding claims, wherein the activation catheter (32, 54) has the capability to carry the implant axially out of the implantation site, once the implant has contracted towards its remembered configuration.

8. The system as claimed in claim 7, wherein the activation catheter (32, 54) is equipped with an implant-capturing element that resists relative axial movement of the implant on the catheter, after the implant has contracted towards its remembered configuration.

9. The system as claimed in claim 8, wherein the capturing element comprises at least one abutment edge on a radially outward-facing part of the catheter (32, 54), down onto which the implant may contract in moving towards its remembered configuration.

## Patentansprüche

1. System, umfassend:
ein Implantat zur Implantation an einer Implantationsstelle innerhalb eines Lumens des Körpers eines Menschen oder Tieres, wobei das Implantat (24, 62) eine Matrix mit einer luminalen Oberfläche, einer abluminalen Oberfläche und einer die Oberflächen trennenden Wanddicke aufweist, wobei das Implantat die Fähigkeit aufweist, sich radial über einen Bereich von Durchmessern auszudehnen und zusammenzuziehen, wobei entsprechende Änderungen in der Querschnittsfläche des Lumens (34, 60) durch die Matrix definiert werden,
wobei das Implantat eine gespeicherte Konfiguration vorweist, in der sich der Durchmesser des Implantats am unteren Ende des Bereichs von Durchmessern befindet, so dass das Implantat aufgefordert wird, seine Konfiguration zu der gespeicherten Konfiguration, d.h. zu einer Konfiguration mit vermindertem Durchmesser, nach dem Auferlegen eines äußeren Reizes auf das Implantat zu ändern, und
wobei das Implantat durch ein Molekül gekennzeichnet ist, das in seinem Schüttvolumen verteilt ist, das vorzugsweise auf ein angelegtes Feld anspricht, das das Volumen durchdringt, indem es Energie mit einer bestimmten Wellenlänge aus dem angelegten Feld absorbiert, wobei das angelegte Feld den äußeren Reiz darstellt und die bevorzugte Reaktion des Moleküls die Temperatur des Implantats erhöht, wobei der Temperaturanstieg als Aufforderung an das Implantat dient, in die gespeicherte Konfiguration zu wechseln; und
einen Selbstschrumpfungs-Aktivierungskatheter (32, 54), der ausgestattet ist, um dem Implantat das angelegte Feld aufzuerlegen, wodurch das Implantat stimuliert wird, sich radial von einer ersten eingesetzten Konfiguration zu seiner gespeicherten Konfiguration zusammenzuziehen,
wobei der Aktivierungskatheter (32, 54) ein Ballonkatheter mit Mitteln zum Verwalten der Temperatur der Oberfläche des Ballons ist, wodurch die Ballonoberfläche wirksam ist, um Wärmeenergie an die luminale Oberfläche der Implantatmatrix zu leiten.

2. System nach Anspruch 1, wobei das Molekül ein Chromophor ist.

3. System nach Anspruch 1, wobei das angelegte Feld das Molekül auffordert, von einer Konfiguration zu einer anderen Konfiguration zu wechseln.

4. System nach einem der vorstehenden Ansprüche, wobei das Implantat aus einem Formgedächtnispolymer hergestellt ist.

5. System nach einem der vorstehenden Ansprüche, bei dem das Implantat ein Stent ist.

6. System nach einem der vorstehenden Ansprüche, wobei der Aktivierungskatheter (32, 54) mit Mitteln ausgestattet ist, um das Implantat radial nach außen zu einer zweiten eingesetzten Konfiguration zu erweitern, die sich durch einen Implantatdurchmesser unterscheidet, der größer als derjenige der ersten eingesetzten Konfiguration ist.

7. System nach einem der vorstehenden Ansprüche, wobei der Aktivierungskatheter (32, 54) die Fähigkeit aufweist, das Implantat axial aus der Implantationsstelle herauszuführen, sobald sich das Implantat zu seiner gespeicherten Konfiguration zusammengezogen hat.

8. System nach Anspruch 7, wobei der Aktivierungskatheter (32, 54) mit einem Implantat-Erfassungselement ausgestattet ist, das einer relativen axialen Bewegung des Implantats auf den Katheter widersteht, nachdem sich das Implantat zu seiner gespeicherten Konfiguration zusammengezogen hat.

9. System nach Anspruch 8, wobei das Erfassungselement mindestens eine Anstoßkante an einem radial nach außen gerichteten Teil des Katheters (32, 54) umfasst, auf die sich das Implantat beim Bewegen zu seiner gespeicherten Konfiguration zusammenziehen kann.

## Revendications

1. Système comprenant :
un implant pour une implantation sur un site d'implantation dans une lumière du corps d'un être humain ou d'un animal, l'implant (24, 62) présentant une matrice avec une surface luminale, une surface abluminale et une épaisseur de paroi séparant lesdites surfaces, l'implant présentant une capacité à se dilater et se contracter radialement à travers une plage de diamètres, avec des modifications correspondantes dans la section transversale de la lumière (34, 60) définie par la matrice,
ledit implant présentant une configuration mémorisée dans laquelle le diamètre de l'implant se situe à l'extrémité inférieure de ladite plage de diamètres, de sorte que l'implant est incité à changer sa configuration vers ladite configuration mémorisée, c'est-à-dire, vers une configuration de diamètre réduit, lors de l'imposition de l'implant à un stimulus extérieur, et
ledit implant étant **caractérisé par** une molécule distribuée dans son volume en vrac qui répond préférentiellement à un champ appliqué qui imprègne ledit volume en absorbant de l'énergie à une longueur d'onde à partir dudit champ appliqué, dans lequel ledit champ appliqué constitue le stimulus extérieur, et la réponse préférentielle de la molécule élève la température de l'implant, l'élévation de température servant à inciter l'implant à changer vers ladite configuration mémorisée ; et
un cathéter d'activation auto-rétrécissant (32, 54) équipé pour imposer à l'implant ledit champ appliqué pour ainsi stimuler l'implant à se contracter radialement d'une première configuration déployée vers sa configuration mémorisée,
dans lequel le cathéter d'activation (32, 54) est un cathéter à ballonnet avec des moyens pour gérer la température de la surface du ballonnet, de sorte que ladite surface de ballonnet est efficace pour conduire l'énergie thermique vers la surface luminale de la matrice de l'implant.

2. Système tel que revendiqué dans la revendication 1, dans lequel ladite molécule est un chromophore.

3. Système tel que revendiqué dans la revendication 1, dans lequel le champ appliqué incite ladite molécule à passer d'une configuration à une autre configuration.

4. Système tel que revendiqué dans l'une quelconque des revendications précédentes, dans lequel l'implant est constitué d'un polymère à mémoire de forme.

5. Système tel que revendiqué dans l'une quelconque des revendications précédentes, dans lequel l'implant est un stent.

6. Système tel que revendiqué dans l'une quelconque des revendications précédentes, dans lequel le cathéter d'activation (32, 54) est équipé de moyens pour dilater l'implant radialement vers l'extérieur vers une seconde configuration déployée qui se distingue par un diamètre d'implant supérieur à celui de la première configuration déployée.

7. Système tel que revendiqué dans l'une quelconque des revendications précédentes, dans lequel le cathéter d'activation (32, 54) a la capacité de transporter l'implant axialement hors du site d'implantation, une fois que l'implant s'est contracté vers sa configuration mémorisée.

8. Système tel que revendiqué dans la revendication 7, dans lequel le cathéter d'activation (32, 54) est équipé d'un élément de capture d'implant qui résiste à un mouvement axial relatif de l'implant sur le cathéter, après que l'implant s'est contracté vers sa configuration mémorisée.

9. Système tel que revendiqué dans la revendication 8, dans lequel l'élément de capture comprend au moins un bord de butée sur une partie du cathéter (32, 54) orientée radialement vers l'extérieur vers le bas, sur laquelle l'implant peut se contracter en se déplaçant vers sa configuration mémorisée.
